(19) Europäisches Patentamt
European Patent Office
Office européen des brevets

(11) EP 0 765 851 B1

(12) # EUROPÄISCHE PATENTSCHRIFT

(45) Veröffentlichungstag und Bekanntmachung des Hinweises auf die Patenterteilung:
**01.12.1999 Patentblatt 1999/48**

(51) Int Cl.[6]: **C07C 37/20**, C07C 39/16

(21) Anmeldenummer: **96114924.2**

(22) Anmeldetag: **18.09.1996**

(54) **Herstellung von 2,2'-Bis(4-hydroxyphenyl)propanen in Gegenwart sulfonat- und mercaptogruppenhaltiger Organopolysiloxane**

Preparation of 2,2'-bis(4-hydroxyphenyl)propanes in the presence of organopolysiloxanes containing sulphonate and mercapto groups

Préparation des 2,2'-bis(4-hydroxyphényl)propanes en présence des organopolysiloxanes contenant un groupe sulfonate ou mercapto

(84) Benannte Vertragsstaaten:
**BE CH DE ES FR GB IT LI NL**

(30) Priorität: **29.09.1995 DE 19536366**

(43) Veröffentlichungstag der Anmeldung:
**02.04.1997 Patentblatt 1997/14**

(73) Patentinhaber: **Degussa-Hüls Aktiengesellschaft**
**60287 Frankfurt am Main (DE)**

(72) Erfinder:
- **Auer, Emmanuel, Dr.**
  **60528 Frankfurt (DE)**
- **Wieland, Stefan, Dr.**
  **63069 Offenbach (DE)**
- **Lansink-Rotgerink, Hans, Dr.**
  **63864 Glattbach (DE)**
- **Jacobsen, Hauke, Dr.**
  **63486 Bruchköbel (DE)**
- **Riedemann, Heike**
  **63766 Mömbris (DE)**

(56) Entgegenhaltungen:
**EP-A- 0 693 470** **FR-A- 2 685 221**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 765 851 B1

Printed by Jouve, 75001 PARIS (FR)

**Beschreibung**

Die Erfindung betrifft die Herstellung von 2,2'-Bis(4-hydroxyphenyl)propanen durch die Umsetzung von entsprechenden Phenolen mit Ketonen in der Gegenwart sulfonat- und mercaptogruppenhaltiger Organopolysiloxane. Beispielhaft sei die Reaktionsgleichung für die Synthese von Bisphenol-A (BPA)angegeben:

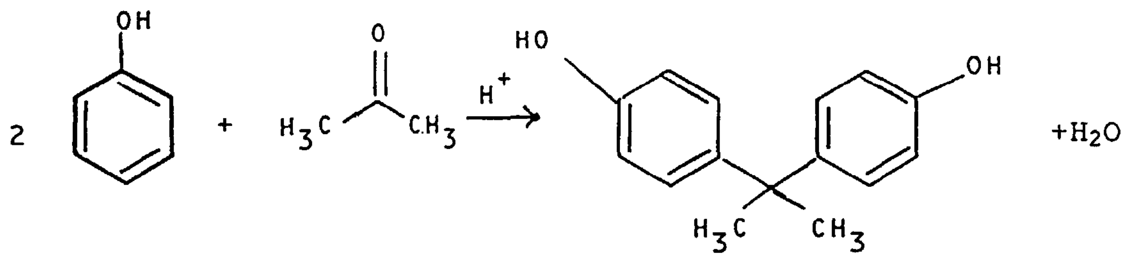

Der homogen mit salzsäure katalysierte Prozeß ist heute weitgehend durch ein Verfahren mit organischen Ionenaustauschern auf Styrol/Divinylbenzolbasis abgelöst worden. Technisch arbeitet man bei dem heterogen katalysierten Verfahren ohne Lösungsmittel in einer 40 - 120°C heißen Schmelze, wobei Phenol und Aceton meist in einem Verhältnis von 10 : 1 bis 15 : 1 eingesetzt werden. Da für die Weiterverarbeitung in Kunststoffen sehr hochreines Bisphenol-A zur Verfügung stehen muß und etwa die Hälfte der bei der Herstellung anfallenden Kosten durch die Reinigung des Produktes verursacht werden, strebt man einen möglichst selektiven Reaktionsverlauf bei hohen Umsätzen an Aceton an. Um Aktivität und Selektivität der heterogenen Katalysatoren zu steigern, verwendet man als Cokatalysatoren bifunktionelle Aminomercaptoverbindungen (z.B. Thiazoline, Mercaptoalkylamine), die über die Amingruppe an den Sulfonsäurerest des organischen Ionenaustauschers gebunden werden (DE-OS 37 27 641, US-PS 3,634,341). Vor Beginn der Reaktion müssen zunächst die sauren Ionenaustauscher in Phenol vorgequollen und anschließend mit den schwefelhaltigen Verbindungen modifiziert werden. Alle möglichen Belegungsgrade der Ionenaustauscher mit Mercaptogruppen sind patentiert worden, doch werden bevorzugt 5 - 30 mol% der Austauschkapazität des Harzes durch Mercaptoverbindungen modifiziert (EP-A1-0 620 041).

Die Herstellung des Bisphenol-A erfolgt in den meisten Fällen über ein kontinuierliches Verfahren, bei dem nach der Reaktion das Bisphenol-A mit Phenol als 1 : 1 Adukt auskristallisiert, von Phenol in verschiedenen Waschprozessen mit Wasser und organischen Lösungsmitteln befreit und als Reinkomponente erneut kristallisiert wird. Die Ausbeuten an Bisphenol-A betragen bei Verweilzeiten am Katalysator von bis zu 6 h zwischen 90 und 95 % bezogen auf die Unterschußkomponente Aceton bei Selektivitäten von 95 %. Das Hauptnebenprodukt ist 2,4'-Dihydroxyphenylpropan (o,p-Bisphenol-A). Weitere, nur in sehr geringer Menge anfallende Verbindungen, wie das 4-Hydroxyphenyl-2,2,4-trimethylchroman oder Triphenole, sind für die gelbe Farbe der Schmelze verantwortlich und ebenfalls unerwünscht.

Allerdings nimmt die Katalysatoraktivität und -selektivität der modifizierten organischen Ionenaustauscher bereits nach relativ kurzer Zeit ab. Eine Ursache ist die Abspaltung der Mercaptoverbindungen von der Oberfläche der sulfonsauren Ionenaustauscher (Desaktivierung der cokatalytischen Einheit), die in verschiedenen Patenten erwähnt wird (EP-A-0 583 712, EP-A1-0 620 041). Der Reaktivitätsverlust der modifizierten Ionenaustauscherharze kann bei Katalysatoren mit geringer Alkyl-SH-Belegung durch Regenerierung teilweise rückgängig gemacht werden (EP-A1-0 620 041). Insgesamt haben die modifizierten Ionenaustauscher im Vergleich zu den nicht modifizierten Systemen bei der Bisphenol-A Synthese jedoch bis zum Faktor 10 kürzere Standzeiten, was notwendige Regenerierungsmaßnahmen erfordert, zu unzureichenden Raum-Zeit-Ausbeuten führt und letztendlich unökonomisch ist. Die Regeneration z. B. großer Mengen Lewatit ist zeitraubend, kostenintensiv und es muß zur Aufrechterhaltung der BPA-Produktion eine gleich große Menge an frischem Ionenaustauscher vorhanden sein (vgl. DE 43 12 039). Außerdem geht aus den genannten Patenten hervor, daß eine häufigere Nachbelegung der organischen Ionenaustauscher mit SH-haltigen Promotoren unter wirtschaftlichen Gesichtspunkten nicht mehr sinnvoll ist, da bei keinem der Regenerierungsschritte die Ausgangsaktivität des Ionenaustauschers erreicht wird, sondern eine kontinuierlich sinkende Aktivität beobachtet wird (DE-A1 43 12 038). Zusätzlich treten im kontinuierlichen Verfahren hydraulische Probleme mit niedrig vernetzten Polystyroldivinylbenzol-Ionenaustauschern aufgrund einer hohen Kompressibilität solcher Harze, damit verbundenem Druckverlust, Kanalbildung im Festbett, Verminderung des Durchsatzes und Einbußen beim Acetonumsatz auf.

Ein weiteres Verfahren zur Herstellung von BPA findet sich in der US-PS 5,315,042. Als Katalysator verwendet man bekannte Kationenaustauscher, wobei man offensichtlich dem Reaktionsgemisch Mercaptoverbindungen als Promotor zusetzt. Bei einer WHSV von 1,0 stellt sich dann ein Acetonumsatz von 81 % ein.

Auch aus der EP-A-0693 470 ist ein Verfahren zur Herstellung von Bisphenol A bekannt, bei dem man einen Polysiloxan-Katalysator einsetzt, der sowohl Sulfonsäureals Auch Mercaptogruppen an der Oberfläche enthält. Das

in dieser Anmeldung beschriebene Herstellverfahren führt zu einem ungeformten Produkt.

Gegenstand der Erfindung ist ein Verfahren zur Herstellung von 2,2'-Bis(4-hydroxyphenyl)propanen durch Umsetzung der entsprechenden Phenole und Carbonylverbindungen in Gegenwart eines sauren Katalysators, das dadurch gekennzeichnet ist, daß man die Reaktion bei einer Temperatur zwischen 40 und 150°C in Gegenwart eines kugelig geformten sulfonat- und mercaptogruppenhaltigen Organopolysiloxans, bestehend aus Einheiten der Formel

$$[O_{3/2}\text{-Si-}R^1\text{-}SO_3\text{-}]_x M^{x+} \quad (I)$$

wobei $R^1$ eine lineare oder verzweigte Alkylengruppe mit 1 bis 12 C-Atomen, eine Cycloalkylengruppe mit 5 bis 8 C-Atomen oder eine Einheit der allgemeinen Formel

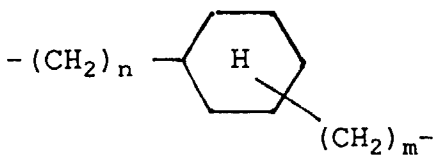 oder 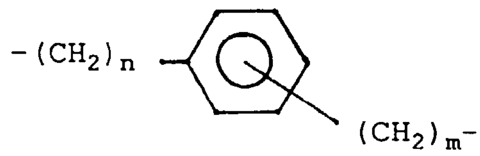 (II)

darstellt, in der n bzw. m eine Zahl von 0 bis 6 ist und die Zahl der silicium- bzw. schwefelständigen Methylengruppen angibt,

M gleich $H^+$ oder gegebenenfalls auch $NH_4^+$ oder ein übliches Metallion wie zum Beispiel ein Alkali oder Erdalkalikation mit einer Wertigkeit von x gleich 1 bis 4 ist,
wobei $H^+$ immer, wenn auch in geringsten Konzentrationen, bevorzugt zu mehr als 1 % der Ionenaustauschkapazität von (I) vorhanden ist,

und Einheiten der Formel

$$O_{3/2}\text{-Si-}R^2\text{-SH} \quad (III)$$

mit $R^2 = R^1$, wobei die freien Valenzen der an das Siliciumatom gebundenen Sauerstoffatome durch die Siliciumatome weiterer Gruppen der Formeln (I) und/oder (II) und/oder durch die Metallatomsauerstoffgruppen der vernetzenden Brückenglieder der Formeln

$$SiO_{4/2} \quad (IV)$$

$$\left.\begin{array}{ll} \text{gegebenenfalls} & R''SiO_{3/2},\ R'SiO_{3/2},\ R'_2SiO_{2/2} \\ \text{und gegebenenfalls} & AlO_{3/2},\ R'AlO_{2/2} \\ \text{und/oder} & TiO_{4/2},\ R'TiO_{3/2},\ R'_2TiO_{2/2} \end{array}\right\} \quad (V)$$

abgesättigt sind,
wobei R' eine Methyl- oder Ethylgruppe ist,
R" Phenyl bedeutet oder eine lineare oder verzweigte $C_2$-$C_{12}$-Alkylgruppe und das Verhältnis der Gruppen der Formel (I) zu Gruppen der Formel (III) von 10 : 1 bis 1 : 10,
das Verhältnis der Gruppen der Formel (I) zu Gruppen der Formel $SiO_{4/2}$ von 1 : 3 bis 1 : 20, und
das Verhältnis der Gruppen der Formel $SiO_{4/2}$ zu Gruppen der Formel (V) von 1 : 0 bis 1 : 0,5 reicht,
durchführt und das gewünschte Produkt abtrennt.

$R^1$ und $R^2$ besitzen dabei in den Formeln (I) und (II) bevorzugt dieselbe Bedeutung,
wobei $R^1$ und $R^2$ insbesondere den Propylenrest und $M^{x+}$ $H^+$ darstellt.

Je nach Vorbehandlung besitzen die kugelig geformten erfindungsgemäß eingesetzten Polysiloxane einen

Teilchendurchmesser von 0,01 bis 3,0, vorzugsweise 0,05 bis 2,0 mm, eine spezifische Oberfläche von > 0 bis 1000, vorzugsweise > 0 bis 700 $m^2/g$ und eine Schüttdichte von 50 bis 1000 g/l, vorzugsweise 100 bis 800 g/l. Die einstellbaren Porendurchmesser liegen im Bereich von > 0 bis über 1000 nm.

Hergestellt werden diese Polysiloxane nach dem in dem in der DE 195 36 363 beschriebenen Verfahren, bei dem man sulfonierte Organosiliciumverbindungen der Formel

$$[(OH)_3Si\text{-}R^1\text{-}SO_3\text{-}]_xM^{x+} \qquad (VI),$$

in der $R^1$, M und x dieselben Bedeutungen wie in Formel (I), haben und bei wäßriger Hydrolyse $SiO_{4/2}$-Einheiten bildende Verbindungen im molaren Verhältnis von 1 : 3 bis 1 : 20 in einem wäßrigen alkoholischen Medium bei 30 bis 100°C mischt und miteinander kondensieren läßt.

Die Umsetzung erfolgt bei Normaldruck oder einem Druck, der der Summe der Partialdrucke der vorhandenen Komponenten bei den eingestellten Temperaturen entspricht. Dieser Mischung fügt man zu Beginn, während oder nach der Kondensationsreaktion, jedoch vor dem Aushärten, eine mercaptogruppenhaltige Organosiliciumverbindung der Formel

$$(RO)_3Si\text{-}R^2\text{-}SH \qquad (VII)$$

in einem molaren Verhältnis zur Verbindung gemäß Formel (VI) von 1 : 10 bis 10 : 1 und gegebenenfalls weitere Gruppen der Formel (V) bildende Verbindungen hinzu.

Der Alkoxyrest von Verbindungen der Formel (VII) entspricht bevorzugt dem verwendeten Alkohol.

Die Reaktionsmischung wird bei einer Temperatur von Raumtemperatur bis 120°C, bevorzugt von 40 bis 80°C, geführt, wobei die Zunahme der Viskosität und die Bildung eines viskosen Gels auftritt. Innerhalb eines Zeitraumes, der unmittelbar nach Zusammengeben aller Reaktionskomponenten bis max. 10 h nach Beginn der Reaktion, jedoch bevorzugt zum Zeitpunkt der Gelbildung liegt, wird die Siloxanlösung in einem mit der Siloxanlösung nicht mischbaren Lösungsmittel dispergiert.

Die mit der Überführung der kohärenten flüssigkeitsdurchsetzten gelartigen Masse in separate sphärische Teilchen einhergehende Formungsphase beginnt mit dem Versetzen der (an)gelierten Reaktionsmischung mit diesem Lösungsmittel in der vorgesehenen Menge.

Die Abtrennung des kugelig geformten feuchten Feststoffs von dem flüssigen Dispersionsmittel kann durch übliche Maßnahmen, wie Dekantieren, Abfiltrieren oder Zentrifugieren erfolgen.

Gegebenenfalls wird der Feststoff nach dem Waschen noch einmal in einer wäßrigen Lösung (die gegebenenfalls HCl enthält) bei einer Temperatur von 90 bis 170°C unter Rühren zur Nachhärtung gegebenenfalls hydrothermal nachbehandelt.

Bevorzugt eingesetzt werden Katalysatoren der Art

$9\ SiO_2 \cdot SiO_{3/2}(CH_2)_3SO_3H \cdot 2\ SiO_{3/2}(CH_2)_3SH$ $10\ SiO_2 \cdot SiO_{3/2}(CH_2)_3SO_3H \cdot 3\ SiO_{3/2}(CH_2)_3SH$ $10\ SiO_2 \cdot 2\ AlO_{3/2} \cdot SiO_{3/2}CH_2\text{-}C_6H_4\text{-}CH_2SO_3H \cdot 2\ SiO_{3/2}(CH_2)_3SH$ $36\ SiO_2 \cdot 4\ SiO_{3/2}(CH_2)_3SO_3H \cdot SiO_{3/2}(CH_2)_3SH$ $9\ SiO_2 \cdot SiO_{3/2}(CH_2)_3SO_3H \cdot 3\ SiO_{3/2}(CH_2)_3SH \cdot SiO_{3/2}(CH_2)_3CH_3$ $6\ SiO_2 \cdot SiO_{3/2}(CH_2)_3SO_3H \cdot 2\ SiO_{3/2}(CH_2)_3SH$

Ein weiterer Gegenstand der Erfindung ist ein Verfahren, das dadurch gekennzeichnet ist, daß man 2,2'-Bis (4-hydroxyphenyl)propan der allgemeinen Formel

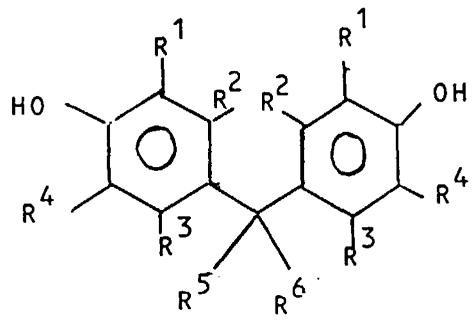

(VIII)

aus Phenolen der allgemeinen Formel

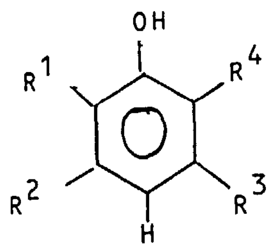

(IX)

in der $R^1$, $R^2$, $R^3$ und $R^4$ unabhängig voneinander für Wasserstoff, $C_1$- bis $C_4$-Alkylgruppen, aromatische Substituenten oder Halogene wie F, Cl, Br und I stehen, bevorzugt, 2-Methylphenol (o-Kresol), 2,6-Dimethylphenol, 2-Ethylphenol, 1,3,5-Trimethylphenol(1,3,5-Xylenol), 2,3,5,6-Tetramethylphenol, 2-Phenylphenol, 2-Chlorphenol, 3-Chlorphenol, 2-Bromphenol, 2,6-Dichlorphenol, insbesondere Phenol, durch Umsetzung mit Carbonylverbindungen der allgemeinen Formel

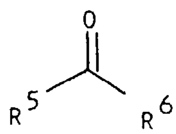

(X)

herstellt, in der $R^5$ und $R^6$ unabhängig voneinander Wasserstoff, $C_1$ bis $C_6$-Alkyl, $C_6$ bis $C_{10}$-Cycloalkyl, $C_3$ bis $C_{14}$-Aryl, $C_7$ bis $C_{20}$-Arylalkyl, $C_7$ bis $C_{20}$-Alkylaryl bedeuten oder $R^5$ und $R^6$ einen gesättigten Ring mit 5 bis 6 Kohlenstoff- oder Heteroatomen bilden, insbesondere die folgenden Aldehyde und Ketone: Formaldehyd, Methylethylketon, Methylpropylketon, Diethylketon, Cyclohexanon, Acetophenon. Bevorzugt wird Aceton verwendet.

Die Kondensation von Phenolderivaten gemäß Formel (IX) mit Carbonylverbindungen gemäß Formel (X) kann kontinuierlich (Festbettreaktor) oder diskontinuierlich (Slurryreaktor) unter den folgenden Bedingungen durchgeführt werden.

Im diskontinuierlichen Betrieb verwendet man im allgemeinen pro Moläquivalent eingesetzter Carbonylverbindung 50 bis 250 g, bevorzugt 100 bis 150 g Trockensubstanz des erfindungsgemäß als Katalysator verwendeten Polysiloxans. Das molare Verhältnis der Ausgangsverbindungen Phenol (I) und Aceton (II) beläuft sich auf 3 : 1 bis 25 : 1, bevorzugt 5 : 1 bis 15 : 1. Die Reaktionstemperaturen liegen im Bereich von 40 bis 150°C, vorzugsweise oberhalb des Schmelz- bzw. Erstarrungspunktes der beteiligten Komponenten. Die Reaktionszeiten bewegen sich im allgemeinen zwischen 10 Minuten und 24 Stunden, bevorzugt zwischen 30 Minuten und 6 Stunden.

Im kontinuierlichen Verfahren wird im allgemeinen ein Gemisch aus Phenolen gemäß Formel (VII) und Carbonylverbindungen gemäß Formel (VIII) im Verhältnis von 3 : 1 bis 25 : 1, bevorzugt 5 : 1 bis 15 : 1 durch einen Festbettreaktor geleitet, der mit bis zu 90 % seines Volumens mit dem erfindungsgemäß einzusetzenden Polysiloxan gefüllt ist. Die Temperatur im Reaktor liegt zwischen 40 und 150°C, bevorzugt zwischen 60 und 75°C. Die WHSV (weight hourly space velocity) bewegt sich im Bereich von 0,25 bis 24 $h^{-1}$, bevorzugt werden zwischen 0,5 und 2 $h^{-1}$. Das Reaktionsgemisch kann dabei den Reaktor von oben nach unten als auch von unten nach oben durchströmen.

Anschließend wird das Produktgemisch der Aufarbeitung und Reinigung zugeführt, die - ebenso wie bei Produktstrom aus dem diskontinuierlich betriebenen Slurryreaktor - durch übliche Methoden wie Destillation und Kristallisation erfolgt. So kann z. B. das hergestellte Bisphenol in der Reaktionsmischung bis zur beginnenden Kristallisation abgekühlt und aus den abfiltrierten Bisphenol-Phenol-Mischkristallen das Phenol durch Destillation oder Extraktion entfernt werden. Das nach diesem Verfahren hergestellte Bisphenol kann ohne Nachreinigung für bekannte Anwendungsgebiete eingesetzt werden.

Die Vorteile der erfindungsgemäß eingesetzten organofunktionellen Polysiloxan-Katalysatoren (OFP) gegenüber dem Stand der Technik sind wie folgt zusammengefaßt:

- Im Vergleich zu nach Beschreibungen aus dem Stand der Technik hergestellten Katalysatoren auf der Basis von Lewatit® und Amberlyst® und zum sonstigen Stand der Tecknik (vgl. US 5,315,042 A) besitzen OFP-Katalysatoren im kontinuierlichen Prozeß höhere Umsatzraten an Aceton bei vergleichbaren WHSV-Werten.
- Auch die Selektivitäten verhalten sich entsprechend: im kontinuierlichen Prozeß liegen die Werte für die Selektivität an p/p-Bisphenol-A bei größer 95,5 % bezogen auf umgesetztes Phenol.
- Die Menge an nicht isomerisierbaren Nebenprodukten liegt bei Verwendung von OFP-Katalysatoren unter 0,05

% (vgl. EP 0 583 712 A2, Selektivität für nicht isomerisierbare Nebenprodukte bei 0,12 % bis 0,27 %).

- Die Selektivitäten für p/p-Bisphenol-A und die isomerisierbaren Produkte o/p-Bisphenol-A im kontinuierlichen Prozeß (WHSV = 0,5 $h^{-1}$) bezogen auf umgesetztes Phenol betragen 99,95 % und mehr.
- Bei Reaktionstemperaturen von 100°C werden im diskontinuierlichen Betrieb nach 4 h Reaktionszeit bei quantitativem Acetonumsatz Ausbeuten an p/p-Bisphenol-A von 97 % erreicht.
- Durch die kovalente Bindung der cokatalytisch wirksamen Mercaptoeinheiten an der Oberfläche der Organofunktionellen Polysiloxanmatrix besitzen derartige Katalysatoren ein hohes Maß an Stabilität gegenüber Desaktivierung, die sich nach dem Stand der Technik durch Auswaschen des Cokatalysators einstellt.
- Außerdem erfordert die kovalente Fixierung der SH-Gruppen weder eine zusätzliche Modifizierung mit Cokatalysatoren vor Beginn der Reaktion, noch den optionalen Zusatz des Cokatalysators in homogener Phase oder die Zugabe geringer Mengen an Wasser zur Reaktionsmischung (vgl. EP 0 583 712 A2), um die Lebensdauer und damit die Standzeit des Katalysators zu erhöhen.
- Organofunktionelle Polysiloxankatalysatoren für die Bisphenol-A Synthese brauchen nicht in Phenol vorzuquellen.
- Sie weisen ein sehr hohes Maß an mechanischer und hydrodynamischer Stabilität auf; deshalb kommt es auch bei hohen Durchsatzraten (Erhöhung der Raum-Zeit-Ausbeuten vgl. DE 43 12 039 A1) nicht zu den beschriebenen hydraulischen Problemen.
- Die OFP-Katalysatoren zeigen eine höhere katalytische Reaktivität pro aktivem Zentrum an der Oberfläche. Im Vergleich zu sulfonsauren organischen Ionenaustauschern weisen die OFP-Katalysatoren eine höhere Reaktionsgeschwindigkeit auf. Diese höhere Zahl der katalytischen Zyklen bei den OFP-Katalysatoren führt insbesondere auch bei hohen Umsätzen der eingesetzten Carbonylverbindungen zu konstant gleichbleibenden Reaktivitäten und einer Verbesserung der Ausbeuten zum Beispiel an Bisphenol-A.

**Beispiel 1**

In einer Rührapparatur werden 10 g eines nach DE 195 36 363 hergestellten und getrockneten Organofunktionellen Polysiloxans der unten angegebenen Zusammensetzung (spezifische Oberfläche: 660 bis 670 $m^2$/g; Gesamtporenvolumen: 2,50 bis 2,60 ml/g; Schüttdichte: 285 bis 290 g/l; Säurekapazität: 0,55 bis 0,75 mmol $H^+$/g Katalysator) mit 70,58 g Phenol und 4,35 g Aceton bei 70°C bis zum vollständigen Acetonumsatz gerührt. Die Ausbeuten an Bisphenol-A werden gaschromatographisch ermittelt.

| Zusammensetzung der Katalysatoren | Ausbeute an p/p-Bisphenol-A | Selektivität bezüglich p/p-Bisphenol-A |
|---|---|---|
| 36 $SiO_2 \cdot 4SiO_{3/2}(CH_2)\text{-}SO_3H \cdot SiO_{3/2}(CH_2)_3SH$ | 80 % | 85,5 % |
| 9 $SiO_2 \cdot SiO_{3/2}(CH_2)_3SO_3H \cdot SiO_{3/2}(CH_2)_3SH$ | 94 % | 94,5 % |
| 9 $SiO_2 \cdot SiO_{3/2}(CH_2)_3SO_3H \cdot 2SiO_{3/2}(CH_2)_3SH$ | 95 % | 95,0 % |
| 9 $SiO_2 \cdot SiO_{3/2}(CH_2)_3SO_3H \cdot 3SiO_{3/2}(CH_2)_3SH$ | 96 % | 95,5 % |

**Beispiel 2**

In einer Rührapparatur werden 20 g eines nach DE 195 36 363 hergestellten und getrockneten Organofunktionellen Polysiloxans der Zusammensetzung 9 $SiO_2 \cdot SiO_{3/2}(CH_2)_3SO_3H \cdot 2\,SiO_{3/2}(CH_2)_3SH$ und den in Beispiel 1 beschriebenen physikalischen Eigenschaften mit 141,16 g Phenol und 8,71 g Aceton bei 100°C gerührt. Man bestimmt gaschromatographisch die Reinheit und die Menge der Produkte.

| Zeit in h | Umsatz an Aceton | Ausbeute an p/p-Bisphenol-A | Selektivität bezüglich p/p Bisphenol-A |
|---|---|---|---|
| 0,5 | 60 % | 57 % | 94 % |
| 2 | 89 % | 87 % | 94 % |
| 4 | 99 % | 97 % | 95 % |

**Beispiel 3**

Ein zylindrischer Reaktor (Durchmesser 28 mm x Länge 170 mm) wird mit 30 g (92 ml) eines nach DE 195 36 363 hergestellten und getrockneten Organofunktionellen Polysiloxans der Zusammensetzung 9 $SiO_2 \cdot SiO_{3/2}(CH_2)_3SO_3H \cdot 2\,SiO_{3/2}(CH_2)_3SH$ und den in Beispiel 1 beschriebenen physikalischen Eigenschaften gefüllt. Man durchströmt den Katalysator mit 3,3 g/min eines Phenol/Aceton-Gemisches im Molverhältnis 10 : 1. Als Wert für die WHSV berechnet sich 6,7 h. Die Temperatur im Festbett beträgt 75°C. Mit Hilfe der Gaschromatographie wird der Umsatz an Aceton zu 41,9 %, die Selektivität für das para/para-Isomere des Bisphenols zu 91,1 % bestimmt. Der Schwefelgehalt im Produktstrom liegt unterhalb von 0,01 mg/g Lösung (kleiner 10 ppm).

**Vergleichsbeispiel 3:**

In einem zylindrischen Reaktor (Durchmesser 28 mm x Länge 170 mm) werden 35 g vorgequollenes, phenolfeuchtes, nach DE-A 36 19 450 bzw. DE-A 37 27 641 mit 25 mol% 2-Mercaptoethylamin modifiziertes, organisches Ionenaustauscherharz vom Typ Lewatit SP 120 ($H^+$-Form) bei 75°C Reaktor-Innentemperatur mit 3,3 ml/min einer Phenol/Aceton-Schmelze im Molverhältnis 10 : 1 durchströmt. Der Wert für die WHSV beträgt 5,7/h. Der Umsatz an Aceton liegt bei 12,5 %, die p/p-Bisphenol-A Selektivität bei 78 %. Der Schwefelgehalt im Produktstrom wird mit 0,061 mg/g Lösung bestimmt.

**Beispiel 4:**

In einem zylindrischen Reaktor (45 mm x 150 mm), der mit 60 g eines nach DE 195 36 363 hergestellten und getrockneten Organofunktionellen Polysiloxans der Zusammensetzung $9\,SiO_2 \cdot SiO_{3/2}(CH_2)_3SO_3H \cdot 2\,SiO_{3/2}(CH_2)_3SH$ und den in Beispiel 1 beschriebenen physikalischen Eigenschaften gefüllt ist, werden 80 g/h eines Phenol/Aceton-Gemisches im Molverhältnis 10 : 1 bei einer Temperatur im Festbett von 75°C durchgeleitet. Das WHSV beträgt 1,3/h. Der Umsatz an Aceton liegt bei 81,0 %, die Selektivität für para/para-Bisphenol-A bei 94,0 %.

**Beispiel 5:**

In einem zylindrischen Reaktor (45 mm x 150 mm), der mit 60 g eines nach DE 195 36 363 hergestellten und getrockneten Organofunktionellen Polysiloxans der Zusammensetzung $9\,SiO_2 \cdot SiO_{3/2}(CH_2)_3SO_3H \cdot 3\,SiO_{3/2}(CH_2)_3SH$ und den in Beispiel 1 beschriebenen physikalischen Eigenschaften gefüllt ist, werden 50 g/h eines Phenol/Aceton-Gemisches im Molverhältnis 10 : 1 bei einer Temperatur im Festbett von 75°C durchgeleitet. Das WHSV beträgt 0,8/h. Man bestimmt mit Hilfe der Gaschromatographie den Umsatz an Aceton zu 95,6 %, die Selektivität für para/para-Bisphenol-A zu 95,4 %.

**Beispiel 6:**

In zwei hintereinander geschalteten, direkt miteinander verbundenen, zylindrischen Reaktoren (45 mm x 150 mm), die mit insgesamt 120 g eines nach DE 195 36 363 hergestellten und getrockneten Organofunktionellen Polysiloxans der Zusammensetzung $9\,SiO_2 \cdot SiO_{3/2}(CH_2)_3SO_3H \cdot 2\,SiO_{3/2}(CH_2)_3SH$ gefüllt sind, werden 60 g/h eines Phenol/Aceton-Gemisches im Molverhältnis 10 : 1 bei einer Temperatur im Festbett von 75°C durchgeleitet. Das WHSV beträgt 0,5/h. Der Umsatz an Aceton liegt bei 98,2 %, die Selektivität für das para/para-Isomere des Bisphenol-A bei 95,5 %, die Selektivität für die Gesamtmenge an Bisphenol-A (Summe aus p/p- und o/p-Produkt) bei 99,95 %.

Zusammenfassung der Ergebnisse im kontinuierlich betriebenen Festbettreaktor Beispiele 3 bis 6:

| WHSV in $h^{-1}$ | Umsatz an Aceton | Ausbeute an p/p-BPA | Selektivität für p/p-BPA | Selektivität für p/p und o/p-BPA |
|---|---|---|---|---|
| 0,5 | 98,2 % | 95,9 % | 95,5 % | 99,95 % |
| 0,8 | 95,6 % | 92,5 % | 95,4 % | 99,31 % |
| 1,3 | 81,0 % | 76,2 % | 94,0 % | 99,02 % |
| 6,7 | 41,9 % | 38,5 % | 91,1 % | 94,11 % |

**Patentansprüche**

1. Verfahren zur Herstellung von 2,2'-Bis(4-hydroxyphenyl)propanen durch Umsetzung der entsprechenden Phenole und Carbonylverbindungen miteinander in Gegenwart eines sauren Katalysators,
dadurch gekennzeichnet,
daß man die Reaktion bei einer Temperatur zwischen 40 und 150°C in Gegenwart eines kugelig geformten sulfonat- und mercaptogruppenhaltigen Organopolysiloxans als Katalysator, bestehend aus Einheiten der Formel

$$[O_{3/2}\text{-}Si\text{-}R^1\text{-}SO_3\text{-}]_x M^{x+} \qquad (I)$$

wobei $R^1$ eine lineare oder verzweigte Alkylengruppe mit 1 bis 12 C-Atomen, eine Cycloalkylengruppe mit 5 bis 8 C-Atomen oder eine Einheit der allgemeinen Formel

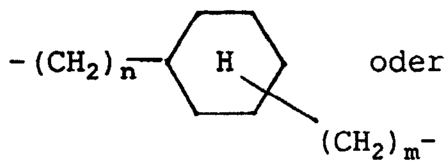 oder 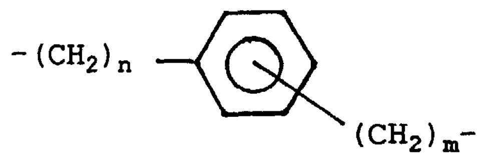 (II)

darstellt, in der n bzw. m eine Zahl von 0 bis 6 ist und die Zahl der silicium- bzw. schwefelständigen Methylengruppen angibt,
M gleich $H^+$ oder gegebenenfalls auch $NH_4^+$ oder ein Metallion mit einer Wertigkeit von x gleich 1 bis 4 ist,
und Einheiten der Formel

$$O_{3/2}\text{-}Si\text{-}R^2\text{-}SH \qquad (III)$$

mit $R^2 = R^1$, wobei die freien Valenzen der an das Siliciumatom gebundenen Sauerstoffatome durch die Siliciumatome weiterer Gruppen der Formeln (I) und/oder (II) und/oder durch die Metallatomsauerstoffgruppen der vernetzenden Brückenglieder der Formeln

$$SiO_{4/2} \qquad (IV)$$

gegebenenfalls $R''SiO_{3/2}$, $R'SiO_{3/2}$, $R'_2SiO_{2/2}$
und gegebenenfalls $AlO_{3/2}$, $R'AlO_{2/2}$
und/oder $TiO_{4/2}$, $R'TiO_{3/2}$, $R'_2TiO_{2/2}$ (V)

abgesättigt sind,
wobei R' eine Methyl- oder Ethylgruppe ist,
R" Phenyl bedeutet oder eine lineare oder verzweigte $C_2$-$C_{12}$-Alkylgruppe und das Verhältnis der Gruppen der Formel (I) zu Gruppen der Formel (III) von 10 : 1 bis 1 : 10,
das Verhältnis der Gruppen der Formel (I) zu Gruppen der Formel $SiO_{4/2}$ von 1 : 3 bis 1 : 20 und
das Verhältnis der Gruppen der Formel $SiO_{4/2}$ zu Gruppen der Formel (V) von 1 : 0 bis 1 : 0,5 reicht,
durchführt und das gewünschte Produkt abtrennt.

2. Verfahren gemäß Anspruch 1,
**dadurch gekennzeichnet,**
daß man ein Gemisch aus Phenolen gemäß der allgemeinen Formel

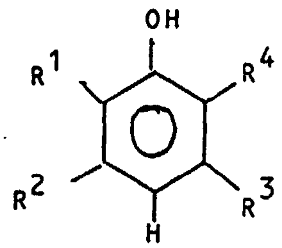

(IX)

in der $R^1$, $R^2$, $R^3$ und $R^4$ unabhängig voneinander für Wasserstoff, $C_1$- bis $C_4$-Alkylgruppen, aromatische Substituenten oder Halogene wie F, Cl, Br und I stehen, durch Umsetzung mit Carbonylverbindungen der allgemeinen Formel

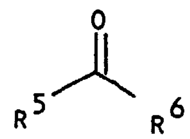

(X)

in der $R^5$ und $R^6$ unabhängig voneinander Wasserstoff, $C_1$ bis $C_6$-Alkyl, $C_6$ bis $C_{10}$-Cycloalkyl, $C_3$ bis $C_{14}$-Aryl, $C_7$ bis $C_{20}$-Arylalkyl, $C_7$ bis $C_{20}$-Alkylaryl bedeuten oder $R^5$ und $R^6$ einen gesättigten Ring mit 5 bis 6 Kohlenstoff- oder Heteroatomen bilden, im molaren Verhältnis von 3 : 1 bis 25 : 1 kontinuierlich durch einen Festbettreaktor leitet.

**3.** Verfahren gemäß den Ansprüchen 1 und 2,
**dadurch gekennzeichnet,**
daß man das Gemisch mit einer WHSV von 0,25 bis 24 $h^{-1}$ durch den Festbettreaktor leitet.

**4.** Verfahren gemäß Anspruch 1,
**dadurch gekennzeichnet,**
daß man das Verfahren diskontinuierlich durchführt, wobei man ein Gemisch aus Phenolen gemäß Formel (IX) gemäß Anspruch 2 und Carbonylverbindungen gemäß Formel (X) gemäß Anspruch 2 im molaren Verhältnis von 3 : 1 bis 25 : 1 in Gegenwart von 50 g bis 250 g Trockensubstanz des Katalysators gemäß Anspruch 1 miteinander umsetzt, bezogen auf ein Moläquivalent der Carbonylverbindung.

**5.** Verfahren gemäß den Ansprüchen 1 bis 4,
**dadurch gekennzeichnet,**
daß das Verhältnis von SH-Gruppen zu $SO_3H$-Gruppen auf dem Katalysator zwischen 1 : 10 und 10 : 1 beträgt.

**6.** Verfahren gemäß den Ansprüchen 1 bis 5,
**dadurch gekennzeichnet,**
daß man ein 2,2'-Bis (4-hydroxyphenyl)propan der allgemeinen Formel

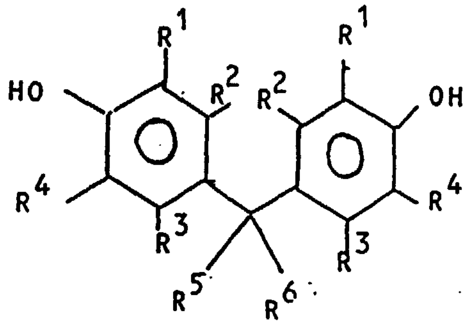

(VIII)

aus Phenolen der allgemeinen Formel

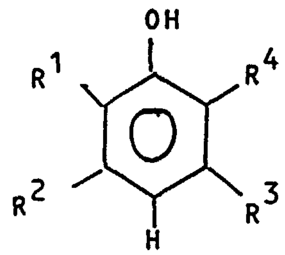

(IV)

in der $R^1$, $R^2$, $R^3$ und $R^4$ dieselbe Bedeutung wie in Anspruch 2 haben durch Umsetzung mit Carbonylverbindungen der allgemeinen Formel

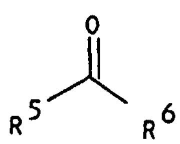

(X)

in der $R^5$ und $R^6$ dieselbe Bedeutung wie in Anspruch 2 haben, gewinnt.

**Claims**

1. Process for the production of 2,2'-bis(4-hydroxyphenyl)propanes by reacting together the corresponding phenols and carbonyl compounds in the presence of an acidic catalyst,
characterised in that
the reaction is performed at a temperature of between 40 and 150°C in the presence of a spherically shaped organopolysiloxane containing sulphonate and mercapto groups as the catalyst, consisting of units of the formula

$$[O_{3/2}\text{-Si-}R^1\text{-}SO_3\text{-}]_x M^{x+} \quad (I),$$

wherein $R^1$ is a linear or branched alkylene group having 1 to 12 C atoms, a cycloalkylene group having 5 to 8 C atoms or a unit of the general formulae

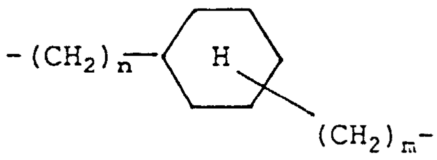 or 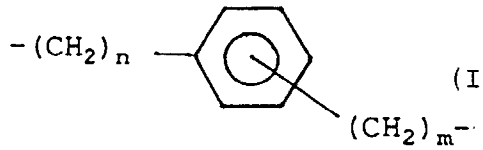 (II)

in which n or m is a number from 0 to 6 and states the number of methylene groups in silicon or sulphur position, M equals $H^+$ or optionally also $NH_4^+$ or a metal ion having a valency of x equals 1 to 4, and units of the formula

$$O_{3/2}\text{-Si-}R^2\text{-SH} \qquad (III)$$

where $R^2 = R^1$, wherein the free valencies of the oxygen atoms attached to the silicon atoms are saturated by the silicon atoms of further groups of the formulae (I) and/or (II) and/or by the metal atom oxygen groups of the crosslinking linking members of the formulae

$$SiO_{4/2} \qquad (IV)$$

$$\left.\begin{array}{ll} \text{optionally} & R''SiO_{3/2},\ R'SiO_{3/2},\ R'_2SiO_{2/2} \\ \text{and optionally} & AlO_{3/2},\ R'AlO_{2/2} \\ \text{and/or} & TiO_{4/2},\ R'TiO_{3/2},\ R'_2TiO_{2/2} \end{array}\right\} \qquad (V)$$

wherein R' is a methyl or ethyl group,
R" means phenyl or a linear or branched $C_2$-$C_{12}$ alkyl group and the ratio of groups of the formula (I) to groups of the formula (III) ranges from 10:1 to 1:10,
the ratio of groups of the formula (I) to groups of the formula $SiO_{4/2}$ ranges from 1:3 to 1:20 and
the ratio of groups of the formula $SiO_{4/2}$ to groups of the formula (V) ranges from 1:0 to 1:0.5,
and the desired product is separated.

**2.** Process according to claim 1,
characterised in that
a mixture of phenols according to the general formula (IX)

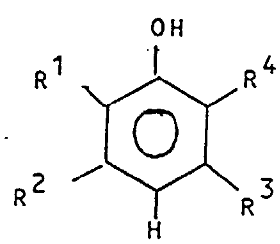 (IX)

in which $R^1$, $R^2$, $R^3$ and $R^4$ mutually independently denote hydrogen, $C_1$ to $C_4$ alkyl groups, aromatic substituents or halogens such as F, Cl, Br and I, by reaction with carbonyl compounds of the general formula

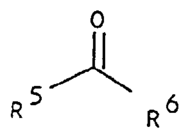

(X)

in which $R^5$ and $R^6$ mutually independently mean hydrogen, $C_1$ to $C_6$ alkyl, $C_6$ to $C_{10}$ cycloalkyl, $C_3$ to $C_{14}$ aryl, $C_7$ to $C_{20}$ arylalkyl, $C_7$ to $C_{20}$ alkylaryl or $R^5$ and $R^6$ form a saturated ring having 5 to 6 carbon atoms or heteroatoms in a molar ratio of 3:1 to 25:1 is continuously passed through a fixed bed reactor.

**3.** Process according to claims 1 and 2,
characterised in that
the mixture is passed through the fixed bed reactor at a WHSV of 0.25 to 24 $h^{-1}$.

**4.** Process according to claim 1,
characterised in that
the process is performed discontinuously, wherein a mixture of phenols according to the formula (IX) according to claim 2 and carbonyl compounds according to the formula (X) according to claim 2 in a molar ratio of 3:1 to 25:1 is reacted in the presence of 50 g to 250 g dry weight of the catalyst according to claim 1, relative to one molar equivalent of the carbonyl compound.

**5.** Process according to claims 1 to 4,
characterised in that
the ratio of SH groups to $SO_3H$ groups on the catalyst is between 1:10 and 10:1.

**6.** Process according to claims 1 to 5,
characterised in that
a 2,2'-bis(4-hydroxyphenyl)propane of the general formula

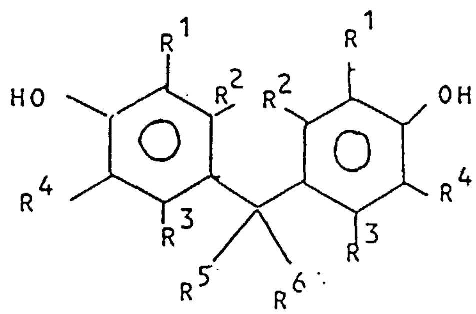

(VIII)

is obtained from phenols of the general formula

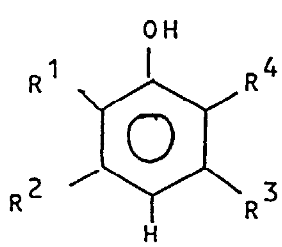

(IV)

in which $R^1$, $R^2$, $R^3$ and $R^4$ have the same meaning as in claim 2 by reaction with carbonyl compounds of the general formula

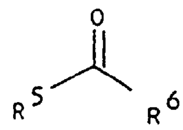

(X)

in which $R^5$ and $R^6$ have the same meaning as in claim 2.

## Revendications

**1.** Procédé de préparation de 2,2'-bis(4-hydroxyphényl)propanes par réaction mutuelle des phénols et des composés de carbonyle correspondants en présence d'un catalyseur acide, caractérisé en ce qu'on effectue la réaction à une température entre 40 et 150°C en présence d'un organopolysiloxane sphérique contenant des groupes sulfonate et mercapto comme catalyseur, constitué d'unités de formule :

$$[O_{3/2}\text{-Si-}R^1\text{-}SO_3\text{-}]_x M^{x+} \qquad (I)$$

dans laquelle $R^1$ représente un groupe alkylène linéaire ou ramifié ayant 1 à 12 atomes de carbone, un groupe cycloalkylène ayant 5 à 8 atomes de carbone ou une unité de formule générale :

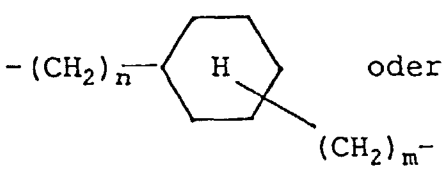

oder

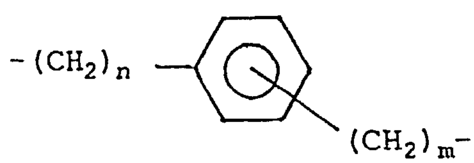

(II)

dans laquelle n ou m est un nombre de 0 à 6 et indique le nombre de groupes méthylène stables au silicium ou au soufre, M est $H^+$ ou éventuellement également $NH_4^+$ ou un ion métallique d'une valence de x égale à 1 à 4, et d'unités de formule :

$$O_{3/2}\text{-Si-}R^2\text{-SH} \qquad (III)$$

dans laquelle $R^2$=$R^1$, les valences libres des atomes d'oxygène liés à l'atome de silicium sont saturées par les atomes de silicium d'autres groupes de formules (I) et/ou (II) et/ou par les groupes oxygène des atomes métalliques des éléments de pontage réticulants de formules :

$$SiO_{4/2} \qquad (IV)$$

éventuellement $R''SiO_{3/2}$, $R'SiO_{3/2}$, $R'_2SiO_{2/2}$

et éventuellement $AlO_{3/2}$, $R'AlO_{2/2}$

et/ou $TiO_{4/2}$, $R'TiO_{3/2}$, $R'_2TiO_{2/2}$ (V)

dans lesquelles R' est un groupe méthyle ou éthyle, R" est un groupe phényle ou un groupe alkyle en $C_2$-$C_{12}$ linéaire ou ramifié et le rapport des groupes de formule (I) aux groupes de formule (III) est de 10:1 à 1:10, le rapport des groupes de formule (I) aux groupes de formule $SiO_{4/2}$ est de 1:3 à 1:20 et le rapport des groupes de formule $SiO_{4/2}$ aux groupes de formule (V) est de 1:0 à 1:0,5, et on sépare le produit souhaité.

**2.** Procédé selon la revendication 1,

caractérisé en ce qu'on fait passer un mélange de phénols de formule générale :

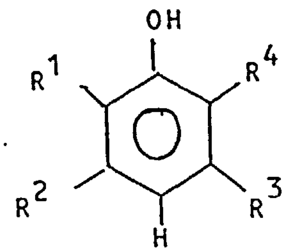

(IX)

dans laquelle $R^1$, $R^2$, $R^3$ et $R^4$ sont, indépendamment les uns des autres, de l'hydrogène, des groupes alkyle en $C_1$-C4, des substituants aromatiques ou des halogènes, tels que F, Cl, Br et I, par réaction avec des composés de carbonyle de formule générale :

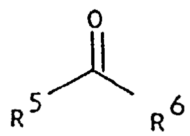

(X)

dans laquelle $R^5$ et $R^6$ désignent, indépendamment l'un de l'autre, de l'hydrogène, un groupe alkyle en $C_1$-$C_6$, cycloalkyle en $C_6$-$C_{10}$, aryle en $C_3$-$C_{14}$, arylalkyle en $C_7$-$C_{20}$, alkylaryle en $C_7$-$C_{20}$ ou $R^5$ et $R^6$ forment un noyau saturé ayant 5 à 6 atomes de carbone ou hétéroatomes, dans le rapport molaire de 3:1 à 25:1, en continu à travers un réacteur à lit fixe.

**3.** Procédé selon les revendications 1 et 2,
caractérisé en ce qu'on fait passer le mélange à travers le réacteur à lit fixe avec une WHSV de 0,25 à 24 $h^{-1}$.

**4.** Procédé selon la revendication 1,
caractérisé en ce qu' on effectue le procédé en discontinu en faisant réagir mutuellement un mélange de phénols de formule (IX) selon la revendication 2 et de composés de carbonyle de formule (X) selon la revendication 2 dans le rapport molaire de 3:1 à 25:1 en présence de 50 g à 250 g de substance sèche du catalyseur selon la revendication 1, par rapport à un équivalent molaire du composé de carbonyle.

**5.** Procédé selon les revendications 1 à 4,
caractérisé en ce que le rapport des groupes SH au groupes $SO_3H$ sur le catalyseur se situe entre 1:10 et 10:1.

**6.** Procédé selon les revendications 1 à 5,
caractérisé en ce qu'on obtient un 2,2'-bis(4-hydroxyphényl)propane de formule générale :

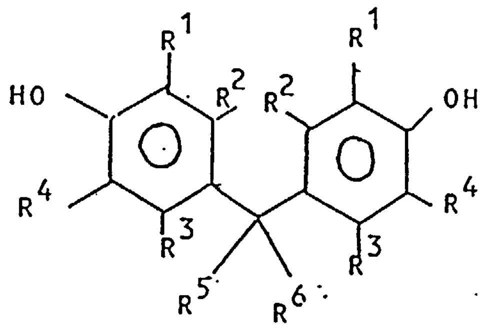

(VIII)

à partir de phénols de formule générale :

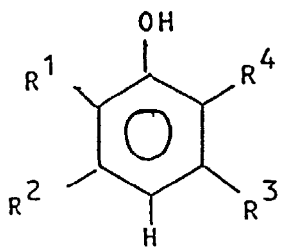

(IV)

dans laquelle $R^1$, $R^2$, $R^3$ et $R^4$ ont la même signification que celle indiquée dans la revendication 2, par réaction avec des composés de carbonyle de formule générale :

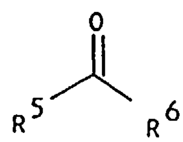

(X)

dans laquelle $R^5$ et $R^6$ ont la même signification que celle indiquée dans la revendication 2.